# EUROPÄISCHE PATENTANMELDUNG

(11) **EP 3 282 249 A1**
(43) Veröffentlichungstag der Anmeldung: **14.02.2018**
(21) Anmeldenummer: 16183334.8
(22) Anmeldetag: 09.08.2016
(51) Int. Cl.: G01N 25/72, G01N 33/44

(54) **VERFAHREN UND VORRICHTUNG ZUR DETEKTION VON GASEN IN KUNSTSTOFFSCHICHTEN DURCH LOCK-IN-INFRAROTTHERMOGRAPHIE**

(71) Anmelder: Saint-Gobain Glass France, 92400 Courbevoie (FR)
(72) Erfinder: PLAHL, Sabrina, 52146 Würselen (DE); LETOCART, Philippe, 4730 Raeren (BE); VORONKOFF, Justine, 92400 Courbevoie (FR)
(74) Vertreter: Lendvai, Tomas

(57) **Zusammenfassung**

Vorrichtung V zur Detektion von Gasen in Kunststoffen durch Lock-in-Infrarotthermographie, umfassend
- eine Lock-in-Infrarotkamera (1) mit
- einem optischen Filter (2) für das Objektiv der Lock-in-Infrarotkamera (1), die mit einer Signalleitungen (7.1) mit
- einem Computer (7) zur Erfassung und Verarbeitung der Signale verbunden ist,
- eine Kunststoffprobe (3),
- eine gepulste Infrarotquelle (4), die mittels einer Signalleitung (6.1) mit
- einer Steuerung (6) der Lock-in-Frequenz verbunden ist.

Verfahren zur Detektion von Gasen in Kunststoffen durch Lock-in-Infrarotthermographie und Verwendung der Vorrichtung und des Verfahrens zur Inline-Qualitätskontrolle bei der Herstellung von Verbundglasscheiben.

## Beschreibung

### Gebiet der Erfindung

Die vorliegende Erfindung betrifft ein Verfahren zur Detektion von Gasen in Kunststoffschichten durch Lock-in-Infrarotthermographie.

Außerdem betrifft die vorliegende Erfindung eine Vorrichtung zur Detektion von Gasen in Kunststoffschichten durch Lock-in-Infrarotthermographie.

### Stand der Technik

Der in der vorliegenden Anmeldung zitierte Stand der Technik wird durch Bezugnahme Bestandteil der Anmeldung.

Ein schwer wiegendes Problem, das häufig bei der Herstellung von Laminaten wie Verbundglasscheiben auftaucht, ist die Bildung von Blasen in Teilen der Laminate nach der Druckbehandlung im Autoklaven oder noch schlimmer bei längerem Gebrauch in der Praxis beim Altern. Durch eine große Menge von Restluft im laminierten Glas entstehen Blasen während und/oder nach der Behandlung im Autoklaven oder während des Wärmetests. Diese Probleme sind weltweit verbreitet und tauchen immer wieder auf. Sie führen zu Ausschuss und damit zu hohen finanziellen Verlusten.

Sensoren, mit deren Hilfe Kohlendioxid oder gelöstes Kohlendioxid bei der Nahrungsmittelherstellung und in der chemischen Industrie detektiert werden kann, sind bereits in Gebrauch. Diese Technologien machen Gebrauch von Infrarot-Wellenlängen, indes werden die Messungen direkt in der Gasphase durchgeführt. Es ist daher nicht möglich, Messungen beispielsweise durch Glas hindurch durchzuführen.

Lock-in-Thermographiekamerasysteme sind bereits im Gebrauch für die Detektion von heißen Stellen und Fehlerstellen in fotovoltaischen Zellen. Diese Systeme können indes nur die Oberfläche scannen, und die getesteten Proben benötigen eine direkte Anregung durch Licht oder elektrische Impulse.

Die Lock-in-Infrarotthermographie kann auch für die thermografische Prüfung von industriellen Gasturbinen verwendet werden. Indes wird auch hier die Einstrahlung von Ultraschall zur Erzeugung von Hotspots benötigt (vgl Sulzer Technical Review 2/2012, 4382).

In der europäischen Patentanmeldung EP 2 840 387 A1 wird eine Anordnung zur Detektion von Hotspots durch Lock-in-Infrarotthermographie beschrieben. Als Energiequelle dient ein in die Probe eingebetteter Stromkreis.

Diese Techniken bieten jedoch keine Lösung für das Problem der Bildung und Detektion von Blasen bzw. zur Detektion von gelösten Gasen.

Es stellt sich daher die Aufgabe, eine Vorrichtung und ein Verfahren zur Detektion von Gasen in Kunststoffen durch Lock-in-Infrarotthermographie zu finden, das die Nachteile des Standes der Technik nicht mehr länger aufweist, sondern rasch und zuverlässig Blasen und gelöste Gase in Kunststoffen detektiert, auch wenn der Kunststoff durch Glas bedeckt ist. Dadurch sollen die Vorrichtung und das Verfahren eine besonders zuverlässige Inline-Qualitätskontrolle beispielsweise bei der Herstellung von Verbundglasscheiben ermöglichen und so den Ausschuss signifikant erniedrigen oder von vornherein vermeiden.

Diese Aufgabe wird durch die Vorrichtung und das Verfahren gemäß den unabhängigen Ansprüchen gelöst. Weitere vorteilhafte Ausführungsformen gehen aus den Unteransprüchen hervor.

Die erfindungsgemäße Vorrichtung zur Detektion von Gasen in Kunststoffen durch Lock-in-Infrarotthermographie umfasst eine Lock-in-Infrarotkamera mit einem optischen Filter für das das Objektiv der Lock-in-Infrarotkamera. Der optische Filter befindet sich an einer beliebigen Stelle innerhalb des optischen Wegs der Lock-in-Infrarotkamera. Die Lock-in-Infrarotkamera ist mit einer Signalleitung mit einem Computer zur Erfassung und Verarbeitung der von der Kamera gelieferten Signale verbunden. Desweiteren umfasst die erfindungsgemäße Vorrichtung eine Kunststoffprobe unterhalb oder oberhalb des optischen Filters. Darunter ist eine gepulste Infrarotquelle angeordnet, die mittels einer Signalleitung mit einer Steuerung der Lock-in-Frequenz verbunden ist. Die gepulste Infrarotquelle kann somit entsprechend der Lock-in-Frequenz moduliert werden.

In einer ersten bevorzugten Ausführungsform umfasst die gepulste Infrarotquelle eine kontinuierliche Infrarotquelle und einen Shutter, der zwischen kontinuierlicher Infrarotquelle und Probe angeordnet ist. Der Shutter moduliert die kontinuierliche Infrarotquelle entsprechend der Lock-in-Frequenz. In dieser Ausführungsform ist der Shutter über eine Signalleitung mit einer Steuerung der Lock-in-Frequenz verbunden, wodurch diese Modulation ermöglicht wird.

In einer zweiten bevorzugten Ausführungsform umfasst die gepulste Infrarotquelle einen pulsbaren thermischen Strahler oder eine Halbleiterquelle. Diese Arten von Quellen sind bereits selbst modulierbar, so dass kein Shutter benötigt wird.

In allen erwähnten Ausführungsformen können zusätzliche optische Bauteile zur Homogenisierung des Beleuchtungsfeldes verwendet werden.

Die erfindungsgemäße Vorrichtung eignet sich für Gase, die IR-Spektren liefern, insbesondere Kohlendioxid. Der optische Filter ist daher vorzugsweise für das IR-Signal von Kohlendioxid bei 2445 cm⁻¹ optimiert. Die Wellenlänge Lambda der Absorption beträgt vorzugsweise 4265 nm und die Bandbreite vorzugsweise 120 nm.

Die Signale, die der Computer von der Lock-in-Infrarotkamera über die Signalleitungen erhält, werden mithilfe einer Software verarbeitet. Ein Beispiel für eine geeignete Software ist PV-LIT® der Firma Infratec.

Die Kunststoffproben sind vorzugsweise Folien, insbesondere Folien, wie sie bei der Herstellung von Verbundglasscheiben üblicherweise verwendet werden.

Vorzugsweise werden die Kunststoffproben aus der Gruppe, bestehend aus Polyvinylbutyral (PVB), Ethylenvinylacetat (EVA), Polyurethan (PU), Polypropylen (PP), Polyacrylat, Polyethylen (PE), Polycarbonat (PC), Polymethylmethacrylat (PMMA), Polyvinylchlorid (PVC), Polyacetatharz, Gießharzen, Polyacrylaten, fluorierten Ethylen-Propylen-Copolymerisaten, Polyvinylfluorid und/oder Ethylen-Tetrafluorethylen-Copolymerisaten, ausgewählt. Insbesondere wird PVB verwendet.

Die Kunststoffproben können von Glas bedeckt sein. Vorzugsweise werden Glasscheiben verwendet, wie sie üblicherweise für die Herstellung von Verbundglasscheiben verwendet werden.

Als Infrarotquellen können unterschiedliche Vorrichtungen verwendet werden, deren Temperaturen vorzugsweise zwischen Raumtemperatur und 240 °C eingestellt werden können.

In einer besonders bevorzugten Ausführungsform werden als kontinuierliche Infrarotquelle heiße Platten in Kombination mit einem Shutter verwendet.

In einer anderen bevorzugten Ausführungsform werden pulsbare thermische Strahler oder Halbleiterquellen in Verbindung mit einer Infratotoptik zur Homogenisierung des Beleuchtungsfeldes eingesetzt.

Die erfindungsgemäße Vorrichtung wird insbesondere für das erfindungsgemäße Verfahren zur Detektion von Gasen in Kunststoffen durch Lock-in-Infrarotthermographie verwendet.

Bevorzugt liefern die Gase IR-Spektren.

Bevorzugt ist das Gas Kohlendioxid.

Bei dem erfindungsgemäßen Verfahren wird die Kunststoffprobe mit Infrarotstrahlung der Infrarotquelle periodisch bestrahlt, wobei die Lock-in-Infrarotkamera die Fluktuation des Grauwertes registriert und wobei die Periodizität durch den Shutter beziehungsweise die Pulsfrequenz der Infrarotquelle bei der Lock-in-Frequenz sichergestellt wird.

Die erhaltenen Signale werden mit einer Software, beispielsweise PV-LIT® der Firma Infratec, die eine temporale Fouriertransformation durchführt, verarbeitet und als Abbildung, worin die Temperaturen als Farbskala wiedergegeben werden, ausgegeben.

Hierbei zeigt es sich, dass die mit Kohlendioxid angereicherten Bereiche der Kunststoffprobe kühler erscheinen als die kohlendioxidfreien Referenzbereiche.

Die blasenfreien transparenten Verbundscheiben, insbesondere die mithilfe der erfindungsgemäßen Vorrichtung und des erfindungsgemäßen Verfahrens selektierten Verbundscheiben, können hervorragend als auf Dauer blasenfreie, bewegliche und unbewegliche, funktionale und/oder dekorative Einzelstücke und/oder als auf Dauer blasenfreie Einbauteile in Möbeln, Geräten und Gebäuden sowie als auf Dauer blasenfreie, transparente Einbauteile in Fortbewegungsmitteln zur Fortbewegung auf dem Lande, in der Luft oder zu Wasser, wie Flugzeuge, Schiffe, Züge und Kraftfahrzeuge, insbesondere aber in Kraftfahrzeugen, beispielsweise als Windschutzscheiben, Heckscheiben und Seitenscheiben und/oder Glasdächer, insbesondere aber als Windschutzscheiben, verwendet werden.

Es versteht sich, dass die vorstehend genannten und nachstehend näher erläuterten Merkmale nicht nur in den angegebenen Kombinationen und Konfigurationen, sondern auch in anderen Kombinationen und Konfigurationen oder in Alleinstellung einsetzbar sind, ohne den Rahmen der vorliegenden Erfindung zu verlassen.

### Kurze Beschreibung der Figuren

Die Erfindung wird nun anhand von Ausführungsbeispielen näher erläutert, wobei Bezug auf die beigefügten Figuren genommen wird. Es zeigen in vereinfachter, in nicht maßstäblicher Darstellung:
- Figur 1a: eine Funktionsskizze der Vorrichtung zur Detektion von Gasen in Kunststoffen durch Lock-in-Infrarotthermographie,
- Figur 1b: eine Funktionsskizze einer weiteren Vorrichtung zur Detektion von Gasen in Kunststoffen durch Lock-in-Infrarotthermographie und
- Figur 2: eine Draufsicht auf die Abbildung der verarbeiteten Signale einer Probe aus Polyvinylbutyral (PVB), die partiell mit Kohlendioxid gesättigt ist.

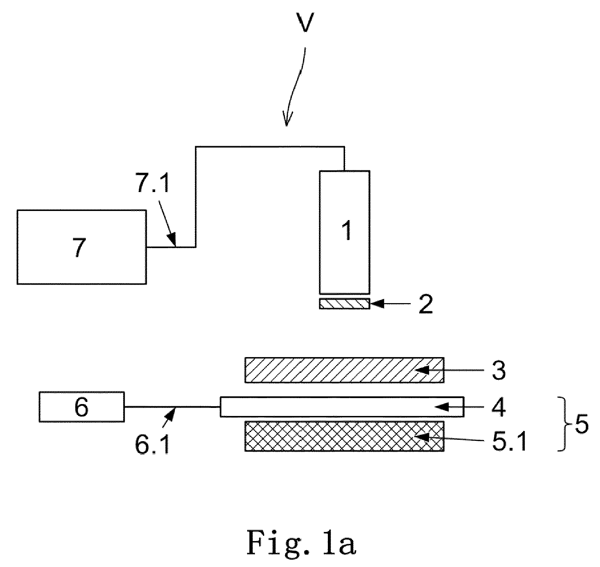

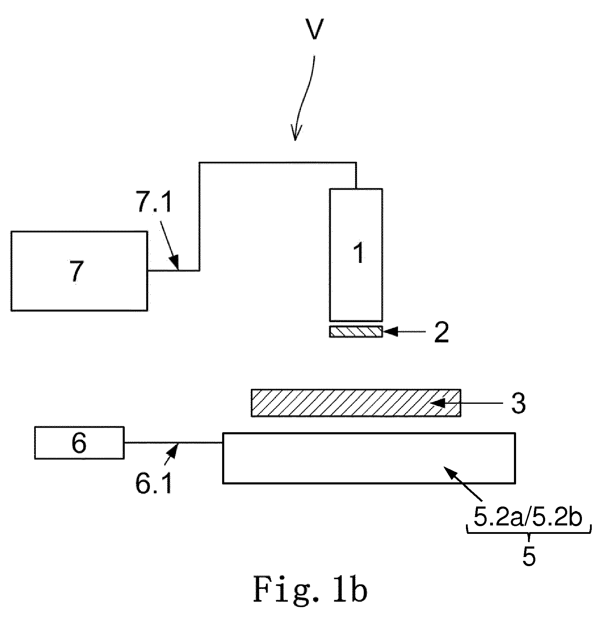

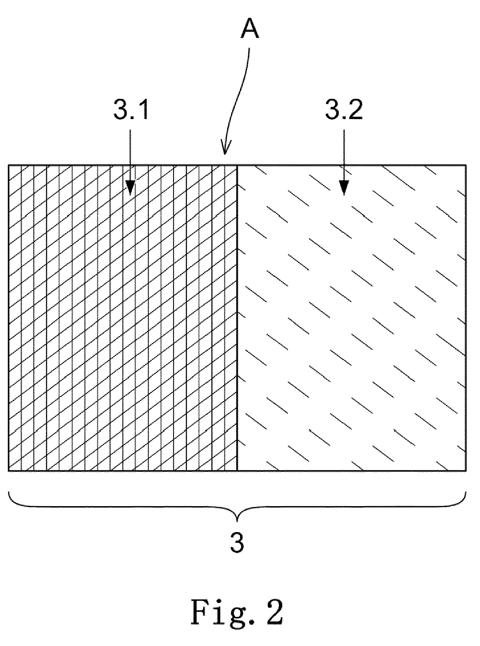

In den Figuren 1a, 1b und 2 haben die Bezugszeichen die folgende Bedeutung:
- A: Abbildung der verarbeiteten Signale
- V: Funktionsskizze einer Vorrichtung zur Detektion von Gasen in Kunststoffen durch Lock-in-Infrarotthermographie
- 1: Lock-in-Infrarotkamera
- 2: optischer Filter für das Objektiv der Kamera 1
- 3: Kunststoffprobe
- 3.1: kohlendioxidfreie Referenzprobe 3
- 3.2: mit Kohlendioxid gesättigte Probe 3
- 4: Shutter
- 5: gepulste Infrarotquelle
- 5.1: kontinuierliche Infrarotquelle
- 5.2a: pulsbarer thermischer Strahler
- 5.2b: Halbleiterquelle
- 6: Steuerung der Lock-in-Frequenz
- 6.1: Signalleitung
- 7: Erfassung und Verarbeitung der Signale
- 7.1: Signalleitung

### Ausführliche Beschreibung der Figuren

### Figuren 1 und 2

Die Figur 1a zeigt eine Funktionsskizze einer ersten Ausführungsform der Vorrichtung V zur Detektion von Gasen in Kunststoffen durch Lock-in-Infrarotthermographie.

Das Objektiv (nicht wiedergegeben) einer Lock-in-Infrarotkamera 1 wurde mit einem optischen Filter 2 ausgestattet. Dazu wurde der optische Filter 2 mit einem Metallring an dem Objektiv befestigt. Die Wellenlänge Lambda der Absorption betrug 4265 nm, die Bandbreite 120 nm. Die Lock-in-Infrarotkamera 1 war über die Signalleitung 7.1 an einen Computer zur Erfassung und Verarbeitung der von der Lock-in-Infrarotkamera 1 erhaltenen Signale angeschlossen.

Die Probe 3 (vorliegend eine PVB-Folie der Abmessungen 30 cm x 30 cm) war unterhalb des optischen Filters 2 angeordnet. Darunter befand sich ein Gerät 6 zur Steuerung der Lock-in-Frequenz über eine Signalleitung 6.1 zum Shutter 4. Unterhalb des Shutters war eine 140°C heiße Platte der Abmessungen 30 cm x 30 cm als kontinuierliche Infrarotquelle 5.1 angeordnet. Die kontinuierliche Infrarotquelle 5.1 und der Shutter 4 ergeben zusammen die erfindungsgemäße gepulste Infrarotquelle 5. Mithilfe der Vorrichtung V konnte die Probe 3 rasch gescannt und die erhaltenen Signale rasch verarbeitet werden.

Die Figur 1 b zeigt eine Funktionsskizze einer zweiten Ausführungsform der Vorrichtung V zur Detektion von Gasen in Kunststoffen durch Lock-in-Infrarotthermographie.

Der Aufbau entspricht im Wesentlichen dem in Figur 1a beschriebenen. Im Unterschied dazu umfasst die gepulste Infrarotquelle 5 gemäß Figur 1b einen pulsbaren thermischen Strahler 5.2a oder eine gepulste Halbleiterquelle 5.2b. In diesem Fall ist die Infrarotquelle über die Signalleitung 6.1 mit dem Gerät 6 zur Steuerung der Lock-in-Frequenz verbunden.

Die Figur 2 zeigt eine Draufsicht auf die Abbildung A der verarbeiteten Signale der Probe 3 aus Polyvinylbutyral (PVB), die bei Raumtemperatur etwa zur Hälfte mit Kohlendioxid gesättigt (Probe 3.2) war. Die Anordnung gemäß Figur 2 zeigt dabei eine Referenzprobe, bei der zur Verdeutlichung des Funktionsprinzips eine Hälfte der Probe vollständig mit Kohlendioxid gesättigt wurde, während die andere Hälfte der Probe keiner Behandlung mit CO₂ unterzogen wurde.

Da das in der Probe 3 gelöste Kohlendioxid die Infrarotstrahlung absorbierte, war die Transmission im Bereich 3.2 geringer als im Referenzbereich 3.1. Dadurch erschien der Bereich 3.2 kühler als der Bereich 3.1.

Die Detektion von gelöstem Kohlendioxid lieferte auch dann hervorragende Ergebnisse, wenn die Probe 3 in einem Verbund aus Glas eingebettet war.

## Patentansprüche

**1.** Vorrichtung V zur Detektion von Gasen in Kunststoffen durch Lock-in-Infrarotthermographie, umfassend
- eine Lock-in-Infrarotkamera (1), die mittels einer Signalleitung (7.1) mit
- einem Computer (7) zur Erfassung und Verarbeitung der Signale verbunden ist,
- einen optischen Filter (2) im optischen Weg der Lock-in-Infrarotkamera (1),
- eine Kunststoffprobe (3),
- eine gepulste Infrarotquelle (5), die
- mittels einer Signalleitung (6.1) mit
- einer Steuerung (6) der Lock-in-Frequenz verbunden ist.

**2.** Vorrichtung V nach Anspruch 1, **dadurch gekennzeichnet, dass** die gepulste Infrarotquelle (5) eine kontinuierliche Infrarotquelle (5.1) und einen Shutter (4), zwischen kontinuierlicher Infrarotquelle (5.1) und Kunststoffprobe (3) umfasst.

**3.** Vorrichtung V nach Anspruch 1 oder 2, **dadurch gekennzeichnet, dass** der Shutter (4) die kontinuierliche Infrarotquelle (5.1) entsprechend der Lock-In-Frequenz moduliert.

**4.** Vorrichtung V nach Anspruch 1, **dadurch gekennzeichnet, dass** die gepulste Infrarotquelle (5) ein pulsbarer thermischer Strahler (5.2a) oder eine Halbleiterquelle (5.2b) ist.

**5.** Vorrichtung V nach einem der Ansprüche 1 bis 4, **dadurch gekennzeichnet, dass** die Wellenlänge Lambda der Absorption des optischen Filters (2) 4265 nm und die Bandbreite des optischen Filters (2) 120 nm betragen.

**6.** Vorrichtung V nach einem der Ansprüche 1 bis 5, **dadurch gekennzeichnet, dass** die Signale in dem Computer (7) mit einer Software verarbeitbar sind.

**7.** Vorrichtung V nach einem der Ansprüche 1 bis 6, **dadurch gekennzeichnet, dass** die Kunststoffprobe (3) eine Folie ist.

**8.** Vorrichtung V nach einem der Ansprüche 1 bis 7, **dadurch gekennzeichnet, dass** die Kunststoffprobe (3) Polyvinylbutyral (PVB), Ethylenvinylacetat (EVA), Polyurethan (PU), Polypropylen (PP), Polyacrylat, Polyethylen (PE), Polycarbonat (PC), Polymethylmethacrylat (PMMA), Polyvinylchlorid (PVC), Polyacetatharz, Gießharze, Polyacrylate, fluorierte Ethylen-Propylen-Copolymerisate, Polyvinylfluorid und/oder Ethylen-Tetrafluorethylen-Copolymerisate, besonders bevorzugt PVB, umfasst.

**10.** Vorrichtung V nach einem der Ansprüche 1 bis 9, **dadurch gekennzeichnet, dass** die Kunststoffprobe (3) von Glas bedeckt ist.

**11.** Verfahren zur Detektion von Gasen in Kunststoffen durch Lock-in-Infrarotthermographie, **dadurch gekennzeichnet, dass** man hierzu eine Vorrichtung V gemäß einem der Ansprüche 1 bis 10 verwendet.

**12.** Verfahren nach Anspruch 11, **dadurch gekennzeichnet, dass** die Probe (3) mit Infrarotstrahlung der gepulsten Infrarotquelle (5) periodisch bestrahlt wird, wobei die Lock-in-Infrarotkamera (1) die Fluktuation des Grauwertes registriert und wobei die Periodizität durch einen Shutter (4), einen pulsbaren thermischen Strahler oder eine Halbleiterquelle sichergestellt wird.

**13.** Verfahren nach Anspruch 11 oder 12, **dadurch gekennzeichnet, dass** die erhaltenen Signale mit einer Software, die eine temporale Fouriertransformation durchführt, verarbeitet werden und als Abbildung, worin die Temperaturen als Farbskala wiedergegeben sind, ausgegeben werden.

**14.** Verfahren nach einem der Ansprüche 11 bis 13, **dadurch gekennzeichnet, dass** mit Kohlendioxid angereicherte Bereiche (3.2) der Probe (3) kühler erschien als die kohlendioxidfreien Referenzbereiche (3.1).

**15.** Verwendung der Vorrichtung V gemäß einem der Ansprüche 1 bis 10 und des Verfahrens gemäß einem der Ansprüche 11 bis 14 zur Inline-Qualitätskontrolle bei der Herstellung von Verbundglasscheiben.
